# EUROPÄISCHE PATENTSCHRIFT

(11) **EP 0 942 999 B1**
(45) Veröffentlichungstag und Bekanntmachung des Hinweises auf die Patenterteilung: **18.05.2005**
(21) Anmeldenummer: 97952888.2
(22) Anmeldetag: 05.12.1997
(51) Int. Cl.: C12N 15/65, C12N 15/85, C12N 5/16, C12Q 1/68

(54) **GENFALLEN-KONSTRUKT ZUR IDENTIFIZIERUNG UND ISOLIERUNG VON GENEN**
GENE TRAP CONSTRUCT FOR IDENTIFICATION AND ISOLATION OF GENES
PIEGE A GENES OBTENU PAR RECOMBINAISON POUR IDENTIFIER ET ISOLER DES GENES

(30) Priorität: 06.12.1996 DE 19650714
(43) Veröffentlichungstag der Anmeldung: 22.09.1999
(73) Patentinhaber: Hölzer, Dieter, 60596 Frankfurt am Main (DE); Von Melchner, Harald, 60590 Frankfurt am Main (DE)
(72) Erfinder: VON MELCHNER, Harald, Universitätsklinikum, D-60596 Frankfurt am Main (DE)
(74) Vertreter: Grund, Martin, Dr.
(86) Internationale Anmeldenummer: PCT/EP1997/006816
(87) Internationale Veröffentlichungsnummer: WO 1998/024918

(56) Entgegenhaltungen:
- EP-A- 0 731 169
- WO-A-97/39722
- SKARNES W C: "THE IDENTIFICATION OF NEW GENES: GENE TRAPPING IN TRANSGENIC MICE" CURRENT OPINION IN BIOTECHNOLOGY, Bd. 4, 1.Januar 1993, Seiten 684-689, XP000569473
- SKARNES W C ET AL: "A GENE TRAP APPROACH IN MOUSE EMBRYONIC STEM CELLS: THE LACZ REPORTER IS ACTIVATED BY SPLICING REFLECTS ENDOGENOUS GENE EXPRESSION AND IS MUTAGENIC IN MICE" GENES AND DEVELOPMENT, Bd. 6, Nr. 6, 1.Juni 1992, Seiten 903-918, XP000569059 in der Anmeldung erwähnt
- HILL D.P. ET AL: "Screening for novel pattern formation genes using *gene* *trap* approaches" METHODS ENZYMOL., 1993, 225/- (664-681), USA, XP002060450 in der Anmeldung erwähnt

## Beschreibung

Die vorliegende Erfindung betrifft ein Genfallen-Konstrukt, das ein erstes Reportergen enthält, das nach Aktivierung ein zweites Reportergen aktivieren kann und die Verwendung dieses Genfallen-Konstrukts für eine Identifizierung und/oder Isolierung von Genen, inbesondere transient exprimierten (transienten) Genen.

Die vorliegende Erfindung betrifft weiterhin eine Zelle, vorzugsweise eine Säugetierzelle, die das oben genannte Genfallen-Konstrukt enthält. Die vorliegende Erfindung betrifft außerdem die Verwendung dieser Säugetierzelle für eine Identifizierung und/oder Isolierung von Genen, insbesondere transienten Genen. Weiterhin betrifft die Erfindung einen Vektor, der das oben genannte Genfallen-Konstrukt enthält, sowie einen Kit zur Identifizierung und/oder Isolierung von Genen, insbesondere transienten Genen, der mindestens das oben genannte Genfallen-Konstrukt oder den oben genannten Vektor enthält.

Schließlich betrifft die vorliegende Erfindung ein Verfahren zur Identifizierung und/oder Isolierung von Genen, insbesondere transienten Genen.

Seit einigen Jahren ist es eines der vorrangigen Ziele der Gentechnologie, Gene zu isolieren und zu identifizieren. Hierfür stehen eine ganze Reihe von Verfahren zur Verfügung, die im wesentlichen darauf gerichtet sind, permanent exprimierte Gene zu isolieren bzw. zu identifizieren.

Sehr viel schwieriger ist die Isolierung und/oder Identifizierung von Genen, die nur transient exprimiert werden, wie z.B. für den programmierten Zelltod verantwortliche Gene, Zellzyklusgene, DNA-Reparaturgene und differenzierungsspezifische Gene.

Um solche Gene in Säugetierzellen zu identifizieren, wo genetische Analysen unter Verwendung von *Drosophila melanogaster* ungeeignet sind, wurde ein Verfahren entwickelt, das auf der Induktion von Genfusionen zwischen einem Reportergen ohne Promotor und den Kontrollelementen eines zellulären Gens durch spezifische Vektoren beruht, die als "Genfallen" oder auch "Promotorfallen" bezeichnet werden. Es sind verschiedene Arten von Vektoren für die Insertionsmutagenese bei Säugetieren entwickelt worden, die ein Reportergen in einer großen Anzahl von zufallsabhängigen Stellen in dem Chromosom inserieren, einschließlich in transkriptionell aktive Bereiche. Indem auf Genexpression selektiert wird, werden Klone erhalten, in denen das Reportergen mit den regulatorischen Elementen eines endogenen Gens fusioniert ist. Auf diese Weise arbeiten die Vektoren als Genfallen und stellen ein sehr hilfreiches Mittel bei der Analyse der Genfunktion dar (Reviews in Hill & Wurst, 1993; Hill & Wurst 1993; von Melchner et al. 1993; von Melchner & Ruley, 1995). In den meisten Fällen werden die Genfallenvektoren als rekombinante Retroviren transduziert, obwohl auch elektroporierte DNA verwendet wurde. Die Retroviren weisen den Vorteil auf, daß sie in weiten Bereichen über das gesamte Genom integrieren, wobei sie die benachbarte DNA kaum schädigen (Varmus, 1988; Coffin et al., 1989; Goff, 1990; Sandmeyer et al., 1990; Withers/Ward et al., 1994).

Es konnte gezeigt werden, daß die Genfallen ein wirksames Mittel für die Analyse der Genfunktionen in Mäusen sind. Indem totipotente Maus-ES-Zellen als zelluläre Ziele verwendet werden, können Maus-Stämme konstruiert werden, die inaktivierte Genfunktionen aufgrund von Mutationen aufweisen. Anders als bei der Genaufspaltung durch homologe Rekombination sind die Genfallen-Verfahren nicht auf Gene beschränkt, für die klonierte Sequenzen erhältlich sind und stellen daher ein Verfahren dar, um noch unbekannte Gene zu isolieren und zu identifizieren.

EP-A-0 731 169 offenbart eine "Genfalle" zur Isolierung und Identifizierung von Genen, die Membranproteine bzw. sezernierte Proteine kodieren. Zu diesem Zweck enthalten die in EP-A-0 731 169 beschriebenen Vektoren ein Fusionskonstrukt, das aus einem Reportergen (β-Galaktosidase-Gen) und einem eine Transmembrandomäne kodierenden Fragment besteht.

Um jedoch Gene zu identifizieren und zu isolieren, die in Zellen erst induziert werden müssen, d.h. nicht ständig transkribiert werden, z.B. transiente Gene, wie z.B. Gene, die für den programmierten Zelltod verantwortlich sind. Zellzyklusgene, DNA-Reparaturgene und differenzierungsspezifische Gene, ist ein zusätzliches Verfahren notwendig, um den in der Genfalle gefangenen zellulären transienten Promotor zuverlässig zu identifizieren, beispielsweise durch ein von der Promotoraktivität unabhängiges, dauerhaftes Signal. Ungefähr 50 % der Gene, die in ES-Zellinien nach einer Infektion/Elektroporation durch Genfallen-Vektoren unterbrochen sind, erzeugen rezessive Phänotypen bei Mäusen (Friedrich und Soriano, 1991; Skarnes et al., 1992; von Melchner et al., 1992). Diese Häufigkeit ist zehnmal größer als die, die nach einer Zufallsinsertion von Retroviren oder mikroinjizierter DNA beobachtet wird (Gridley et al., 1987; Jänisch et al., 1985). Basierend auf dieser hohen Effizienz der Geninaktivierung erscheint es sinnvoll, Zellinien zu isolieren, die Integrationen in den meisten exprimierten Genen (2-4 x 10⁴) aufweisen. Es ist jedoch wenig praktisch, alle Mutationen in die Stammlinie zu übertragen; außerdem betreffen viele Mutationen Gene von geringerer Bedeutung. Deshalb wäre es wünschenswert, mutagenisierte ES-Zellklone für Mutationen auszuwählen, die wichtige biologische Prozesse oder Gene betreffen.

Im Hinblick darauf sind besonders diejenigen Strategien geeignet, die eine Vorauswahl von ES-Zellklonen auf interessante Mutationen in vitro involvieren, und dafür ein Reportergen verwenden, um Mutationen in z.B. differenzierungsspezifischen Genen zu identifizieren. Obwohl gezuchtete ES-Zellen prinzipiell Gene exprimieren könnten, die in vivo nicht exprimiert werden, wurden in 12 Fällen Fusionsgene gefunden, die sowohl in ES-Zellen als auch in Embryos exprimiert wurden (De Gregory et al., 1994; Reddy et al., 1992). Es scheint weiterhin so zu sein, daß ein Wechsel in der Expression von Fusionsgenen während der Differenzierung in vitro sehr genau den Wechsel in der Expression während der in vivo-Entwicklung vorhersagt (Reddy et al., 1992). Dies stimmt mit früheren Beobachtungen überein, bei denen in vitro-regulierte Gene auch in vivo streng reguliert waren (Muthuchamy et al., 1993; Rappotee et al., 1988; Rogers et al., 1991; Scholer et al., 1990; Sharpe et al., 1990).

Es war daher eine Aufgabe gemäß der vorliegenden Erfindung, ein Genfallen-Konstrukt bereitzustellen, das eine Isolierung und Identifizierung von Genen, insbesondere transienten Genen, in besonders vorteilhafter Weise ermöglichte. Es war eine weitere Aufgabe gemäß der vorliegenden Erfindung, eine Zelle anzugeben sowie einen Vektor, die dieses Genfallen-Kostrukt enthalten, sowie einen Kit zur Identifizierung und/oder Isolierung von Genen.

Schließlich war es eine Aufgabe gemäß der vorliegenden Erfindung, ein Verfahren zur Identifizierung und/oder Isolierung von Genen, insbesondere transienten Genen, anzugeben.

Diese Aufgaben werden gelöst durch die Gegenstände der unabhängigen Ansprüche 1, 14 und 20, den Ansprüchen auf eine Zelle 16, einen Vektor, 22, einen Kit, 23, und den Verfahrensanspruch 24.

Bevorzugte Ausführungsformen gemäß der vorliegenden Erfindung sind in den Unteransprüchen angegeben.

Ein "Reportergen" bedeutet hierin eine Nukleinsäuresequenz, die bei Transkription ein nachweisbares Signal, wie beispielsweise ein Proteinprodukt, erzeugt. Das "erste" Reportergen wird durch zelluläre Transkriptionssignale reguliert, in deren Nachbarschaft es integriert wird. Ist das erste Reportergen einmal aktiviert, führt das erste Reportergenprodukt zur Aktivierung des "zweiten" Reportergens, wobei diese zweite Aktivierung dauerhaft, d.h. unabhängig von der Transkriptionsaktivität der das erste Reportergen kontrollierenden DNA-Region ist. Das zweite Reportergen kodiert für ein nachweisbares Genprodukt, wie beispielsweise ein Enzym, das durch eine Farbreaktion nachweisbar ist, oder einen Wachstumsfaktor, auf den hin selektioniert werden kann. Die Kombination aus erstem und zweitem Reportergen führt dazu, daß auch ein nur transient exprimierter Promotor mittels eines dauerhaften Signals (dem zweiten Reportergenprodukt) nachgewiesen werden kann. Das erste Reportergen und das zweite Reportergen können in einem gemeinsamen Konstrukt vorliegen oder sie können auf verschiedenen Konstrukten, die an verschiedenen Orten in der Zelle vorliegen können, vorhanden sein. Das erfindungsgemäße Genfallenkonstrukt ist somit vom Prinzip her eine Kombination aus einem herkömmlichen Genfallen-Konstrukt, wie beispielsweise in US 5364783 beschrieben, das dem ersten Reportergenkonstrukt entspricht, und einem weiteren Konstrukt, das ein dauerhaftes Signal unabhängig von der Promotoraktivität erzeugen kann, im vorliegenden Fall das zweite Reportergenkonstrukt.

Gemäß Anspruch 1 der vorliegenden Erfindung wird ein Genfallen-Konstrukt vorgesehen, das ein erstes Reportergen enthält, das nach Aktivierung ein zweites Reportergen aktivieren kann. Das erste Reportergen kodiert für eine Rekombinase, wobei diese Rekombinase besonders bevorzugt Cre oder Flp ist.

Das zweite Reportergen kodiert für einen Proteinfaktor, in einer weiteren bevorzugten Ausführungsform kodiert das zweite Reportergen für IL-3 und in einer besonders bevorzugten weiteren Ausführungsform für *E. coli* β-Galaktosidase (lacZ).

Das zweite Reportergen wird dadurch aktiviert, daß die Rekombinase ein vor dem zweiten Repörtergen gelegenes DNA-Fragment deletiert und das zweite Reportergen somit unmittelbar stromabwärts von einem Promotor dauerhaft unter dessen Kontrolle plaziert.

Das deletierte DNA-Fragment kann vorzugsweise ein Antibiotika-Resistenzgen sein, wobei das deletierte DNA-Fragment in einer besonders bevorzugten Ausführungsform für Thymidin-Kinase-Neomycin-Phosphotransferase-Fusionsprotein kodiert und in einer weiteren besonders bevorzugten Ausführungsform für eine Neomycinphosphotransferase kodi ert.

Der Promotor, vor den das zweite Reportergen plaziert wird, ist vorzugsweise der Phosphoglyceratkinase-Promotor.

In einer bevorzugten Ausführungsform wird das deletierte DNA-Fragment von Targetsequenzen für die Rekombinase flankiert, die z.B. loxP oder frt sein können. Eine Möglichkeit besteht auch darin, daß sich die Targetsequenzen in der U3- und/oder U5-Region befinden.

Das oben beschriebene Genfallen-Konstrukt kann für eine Identifizierung und/oder Isolierung von Genen, insbesondere transienten Genen, verwendet werden. Hierunter fällt besonders die Isolierung und/oder Identifizierung von für den programmierten Zelltod verantwortlichen Genen, von Zellzyklusgenen, DNA-Reparaturgenen und differenzierungsspezifischen Genen.

Die vorliegende Erfindung sieht außerdem eine Zelle, vorzugsweise eine Säugetierzelle, vor, die ein Genfallen-Konstrukt wie oben beschrieben enthält, wobei es sich nicht um eine totipotente menschliche Stammzelle handelt.

In einer bevorzugten Ausführungsform ist die Säugetierzelle von IL-3 abhängig, und enthält ein Genfallen-Konstrukt, in dem das erste Reportergen für Cre-Rekombinase kodiert, wobei die Cre-Rekombinase ein vor dem zweiten Reportergen gelegenes DNA-Fragment deletieren kann, wobei das deletierte Fragment für ein Thymidinkinase-Neomycinphosphotransferase-Fusionsprotein kodiert und von loxP-Targetsequenzen flankiert ist und wobei das zweite Reportergen für IL-3 kodiert und nach Deletion des Thymidin-Kinase-Neomycinphosphotransferase-Fusionsproteingens unmittelbar stromabwärts des Phosphoglyceratkinasepromotors zu liegen kommt.

Besonders bevorzugt ist die Säugetierzelle eine wachstumsfaktor-abhängige hämatopoetische Vorläuferzelle (z.B. FDCP1) oder eine totipotente nicht-menschliche embryonale Stammzelle.

Die Säugetierzelle gemäß der vorliegenden Erfindung kann für eine Identifizierung und/oder Isolierung von Genen, insbesondere transienten Genen, verwendet werden, wobei insbesondere eine Identifizierung und/oder Isolierung von für den programmierten Zelltod verantwortlichen Genen, von Zellzyklusgenen, DNA-Reparaturgenen und differenzierungsspezifischen Genen bevorzugt ist.

Weiterhin sieht die vorliegende Erfindung einen Vektor vor, der das Genfallen-Konstrukt wie oben beschrieben enthält.

Ferner wird ein Kit zur Identifizierung und/oder Isolierung von Genen vorgestellt, insbesondere transienten Genen, der mindestens ein Genfallen-Konstrukt wie oben beschrieben enthält.

Vorzugsweise enthält der Kit zwei Konstrukte, wobei das erste Konstrukt das erste Reportergen und das zweite Konstrukt das zweite Reportergen enthält.

Der Kit kann auch einen Vektor wie oben beschrieben enthalten.

Schließlich sieht die vorliegende Erfindung ein Verfahren zur Identifizierung und/oder Isolierung von Genen, insbesondere transienten Genen vor, das die folgenden Schritte enthält:
- Einbau eines Genfallen-Konstrukts wie oben beschrieben in eine geeignete Zelle;
- Selektion auf Zellen, bei denen das erste Reportergen in ein Gen, vorzugsweise nicht aktives Gen, eingebaut ist;
- Aktivierung des ersten Reportergens, vorzugsweise durch Initiation der Transkription des nicht aktiven Gens, woraufhin das zweite Reportergen aktiviert wird; und
- Identifizierung und/oder Isolierung des Gens, in das das erste Reportergen eingebaut ist.

In einer bevorzugten Ausführungsform ist das oben beschriebene Verfahren dadurch gekennzeichnet, daß
- ein Genfallen-Konstrukt, wie oben beschrieben, als erstes Reportergen das Cre-Rekombinase-Gen enthält, daß die Cre-Rekombinase ein stromaufwärts vom zweiten Reportergen gelegenes Thymidinkinase-Neomycinphosphotransferase-Gen deletiert, wodurch das zweite Reportergen, das für IL-3 kodiert, neben einem Phosphoglyceratkinase-Promotor zu liegen kommt,
- das Genfallen-Konstrukt in eine IL-3-abhängige FDCP-1-Zelle eingeschleust wird,
- durch Zucht in Neomycin-(z.B. G 418-)haltigem Medium auf Zellen selektiert wird, die Cre-Rekombinase nicht produzieren,
- diese selektierten Zellen in IL-3-freiem Medium gezüchtet werden, so daß für den programmierten Zelltod verantwortliche Gene aktiviert werden,
- daß die überlebenden Zellen selektioniert werden und
- die für den programmierten Zelltod verantwortlichen Gene mit den bekannten Verfahren isoliert werden.

In einer weiteren bevorzugten Ausführungsform ist das Verfahren dadurch gekennzeichnet, daß
- ein Genfallen-Konstrukt, wie oben beschrieben, als erstes Reportergen das Cre-Rekombinase-Gen enthält, daß die Cre-Rekombinase ein stromaufwärts vom zweiten Reportergen gelegenes Neomycinphosphotransferase-Gen deletiert, wodurch das zweite Reportergen, das für lacZ kodiert, neben einem Phosphoglyceratkinase-Promotor zu liegen kommt,
- das Genfallen-Konstrukt in eine totipotente nicht-menschliche embryonale Stammzelle eingeschleust wird,
- durch Anzucht auf G418 auf Zellen selektiert wird, die Cre-Rekombinase nicht produzieren,
- diese selektiven Zellen zum Differenzieren induziert werden, so daß differenzierungsspezifische Gene aktiviert werden,
- diese Zellen isoliert werden,
- diese Zellen in die Keimbahn eines nicht menschlichen Säugetiers eingeführt werden und dort für die Bildung von Geweben verantwortlich sind und
- die differenzierzungsspezifischen Gene mit den bekannten Verfahren isoliert werden.

Das oben genannte Verfahren zur Isolierung von für den programmierten Zelltod verantwortlichen Genen ist deshalb besonders geeignet, weil durch permanenten Selektionsmarkeraustausch durch eine Genfalle, die Cre-Rekombinase exprimiert, im wesentlichen sterbende Zellen durch Aktivierung der endogenen Cytokinproduktion in sich vermehrende Zellen umgewandelt werden. Dies erlaubt eine Selektion für die Integration in Gene, die während des programmierten Zelltods aktiv sind. Die Expression des Selektionsmarkers, z.B. IL-3, wird entkoppelt von der Expression des lokalisierten Gens (in der Genfalle), indem die Rekombinase den Selektionsmarker hinter den Phosphoglyceratkinasepromotor bringt. Dies ist besonders wichtig, da es unwahrscheinlich ist, daß Zellen die für den Zelltod verantwortlichen Gene ständig exprimieren. Schließlich ist das Verfahren gemäß der vorliegenden Erfindung nicht auf hochexprimierte Gene beschränkt, und die Genrepräsentation ist sehr viel gleichmäßiger als z.B. bei der cDNA-Klonierung. Weiterhin ist die Verwendung von Genfallen zur Isolierung und/oder Identifizierung, anders als die differentielle RT-PCR-Amplifikation, gut reproduzierbar, vermeidet eine Redundanz und ermöglicht sogar eine Quantifizierung. Schließlich sind die Genfallen-Proviren ständig in oder nahe der 5'-Exons lokalisiert und haben dieselbe transkriptionelle Orientierung in bezug auf das Gen, was das Klonieren deutlich erleichtert.

Auf jeden Fall ermöglicht die Charakterisierung der zellulären Transkripte, die durch den IL-3-Entzug induziert werden, tiefere Einblicke in die komplexen molekularen Veränderungen, die während des programmierten Zelltods stattfinden.

Bei einer weiteren bevorzugten Ausführungsform enthält eine nicht-menschliche totipotente Zelle, die von sich aus einen gesamten Organismus bilden kann, ein Konstrukt, bei dem das erste Reportergen eine Cre-Rekombinase kodiert, die ein vor dem zweiten Reportergen gelegenes Fragment deletieren kann, wobei das deletierte Fragment für neo kodiert und von loxP-Targetsequenzen flankiert ist, und das zweite Reportergen kodiert für lacZ, das nach Deletion von neo unmittelbar stromabwärts von pgk zu liegen kommt.

Die beigefügten Figuren sollen die vorliegende Erfindung verdeutlichen:

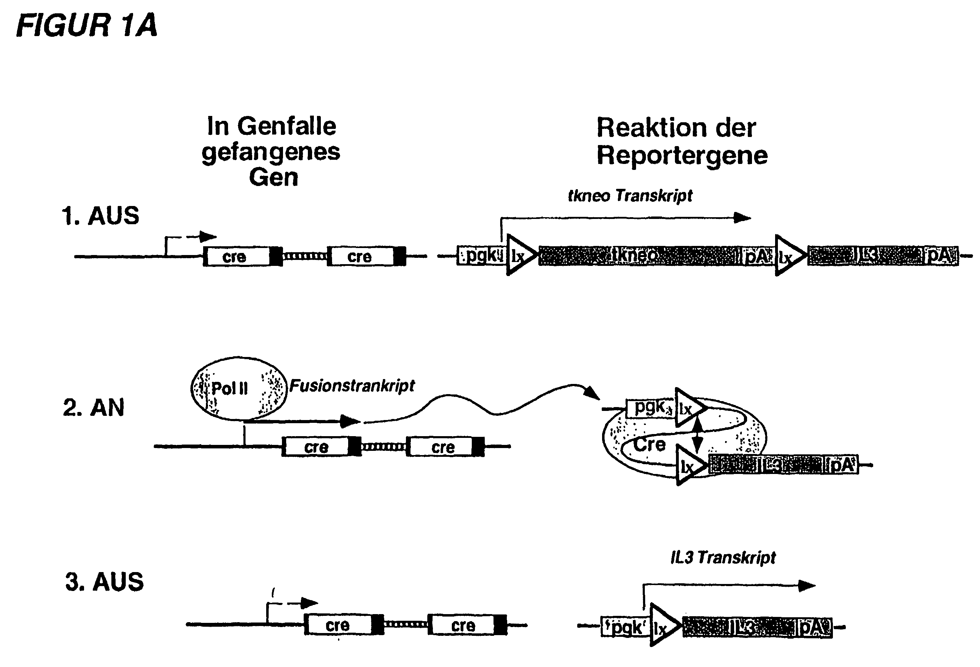

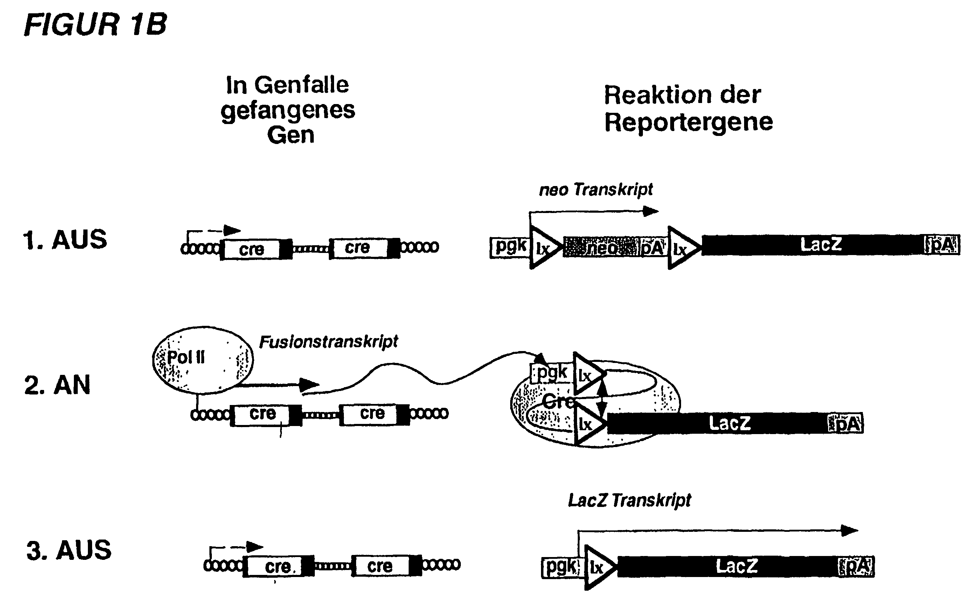

Figur 1 zeigt das Verfahren gemäß der Erfindung in einer schematischen Darstellung zweier bevorzugter Ausführungsformen.
**(A)** Aktivierungsmechanismus der endogenen IL-3 Sekretion in FLOXIL-3-Zellen nach IL-3-Entzug. Eine U3-Cre-Genfallenintegration in ein ursprünglich nicht exprimiertes Apoptosegen wird transient durch IL-3-Entzug aktiviert (li.). Durch ortsspezifische Rekombination des Reporterplasmids ppgklxtkneolL3 wird das tkneo-Gen eliminiert und das IL3-Gen wird unmittelbar stromabwärts des pgk-Promotors plaziert. Dadurch kommt es zu einer anhaltenden IL-3 Synthese, die auch nach Abschaltung des in der Genfalle gefangenen Gens anhält.
**(B)** Aktivierungsmechanismus des LacZ-Gens während der Differenzierung von totipotenten embryonalen Stammzellen -pln13-. Eine U3-Cre-Genfallenintegration in ein ursprünglich nicht exprimiertes Differenzierungsgen wird transient während der Differenzierung aktiviert. Durch ortsspezifische Rekombination des Reporterplasmids ppgklxneoLacZ wird das LacZ-Gen unmittelbar stromabwärts des pgk-Promotors plaziert. Dadurch kommt es zu einer anhaltenden Synthese von β-Galaktosidase, die auch nach Abschaltung des in der Genfalle gefangenen Gens anhält.

Figur 2 zeigt eine schematische Übersicht über ein Verfahren zur Isolierung und Analysierung von induzierbaren Differenzierungsgenen aus embryonalen Stammzellen.

Figur 3 zeigt die Cre/loxP-vermittelte Rekombination des ppgklxtkneoIL3-Plasmids in FLOXIL-3-Zellen mit U3-Cre-Genfallenintegrationen in exprimierten Genen.
**(A)** Struktur von ppgklxtkneoIL3 vor und nach Rekombination.
**(B)** Southern-Blot-Analyse von autonomen und parentalen FLOXIL-3-Zellen. Genomische DNAs wurden mit BamHI geschnitten, in Agarosegelen fraktioniert und auf Nylon-Membranen transferiert. Die Hybridisierung erfolgte mit ³²P-markierten neo-(li.) oder pgk-spezifischen Sonden. Bahn 1:parentale FLOXIL-3-Zellen; Bahnen 2-6: autonome Klone 1-5. Molekulargewichte wurden anhand einer 1 kb-BRL-Leiter bestimmt.

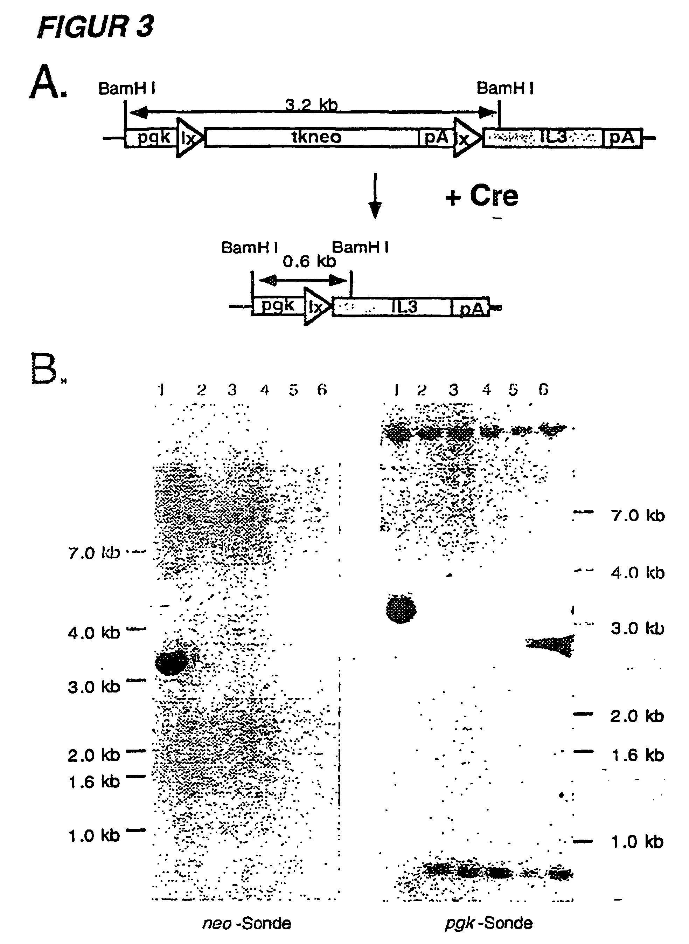

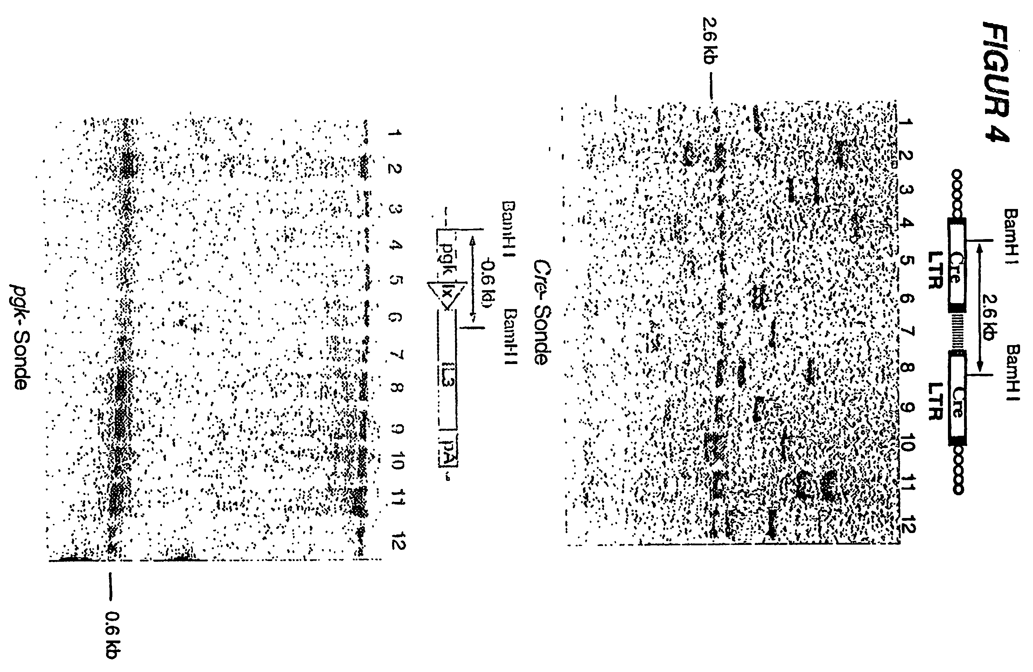

Figur 4 zeigt U3-Cre-Genfallenintegrationen und ortspezifische Rekombination in autonomen, nach IL-3-Entzug isolierten Klonen.
**(Oben)** Erwartete Struktur der U3-Cre-Proviren (li.) und der rekombinierten Reporterplasmide (re.).
**(Unten)** Southern-Blot-Analyse von Klonen, die nach IL-3-Entzug aus der U3-Cre/FLOXIL-3-Integrationsbank isoliert wurden. Die genomische DNA der einzelnen Klone wurde mit BamHI geschnitten und wie in der Legende zu Figur 3 behandelt. Die Hybridisierung erfolgte mit ³²P-markierten Cre- (links) oder pgk-(rechts) spezifischen Sonden. Bahn 1,26-11-1; Bahn 2,26-11-3. Bahn 3,26-11-4; Bahn 4,26-11-5; Bahn 5,26-11-6, Bahn 6,26-11-7; Bahn 7,26-11-8. Bahn 8,26-12-1; Bahn 9,26-12-2; Bahn 10,26-12-3; Bahn 11,26-12-4; Bahn 12,26-12-5. Die abgebildeten Proben sind für die gesamte Integrationsbank repräsentativ.

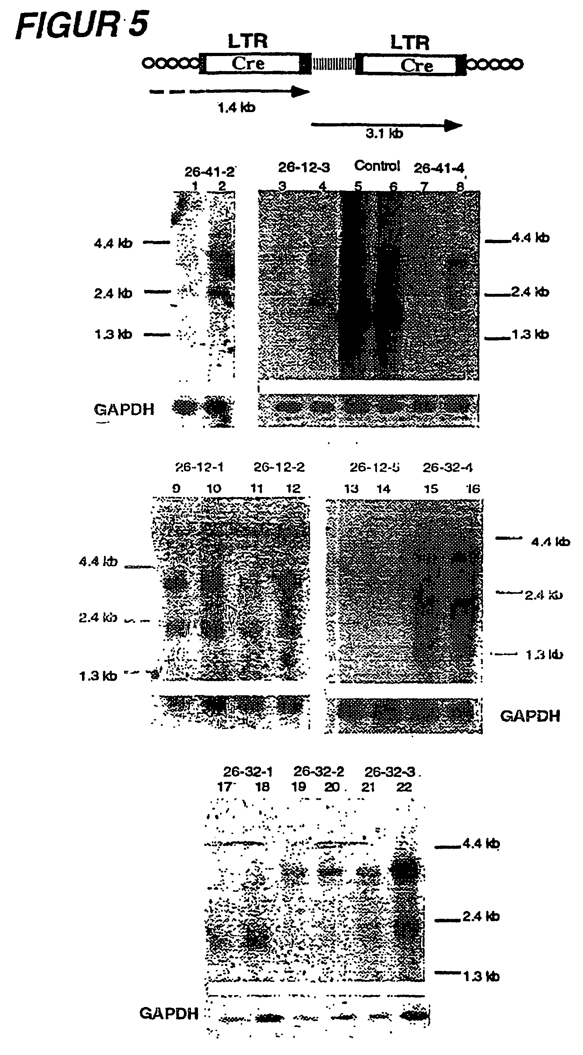

Figur 5 zeigt eine Untersuchung der Zell-Provirus-Fusionstranskripte.
**(Oben)** Von U3-Cre-Genfallenintegrationen in exprimierte Gene erwartete Transkripte.
**(Unten)** Northern-Blot-Analyse von Zell-Provirus-Fusionstranskripten vor und nach Serumentzug. Polyadenylierte RNA (5 µg) wurde in Formaldehyd-Agarose-Gelen fraktioniert und auf Nylonmembranen transferiert. Die Hybridisierung erfolgte mit ³²P-markierten Cre- oder GAPDH-spezifischen Sonden. Ungerade Zahlen repräsentieren RNAs vor, gerade Zahlen RNAs nach 16-stündigem Serumentzug. Bahnen 5 und 6 enthalten RNA von einem Klon mit einer konstitutiv exprimierten U3-Cre-Integration.

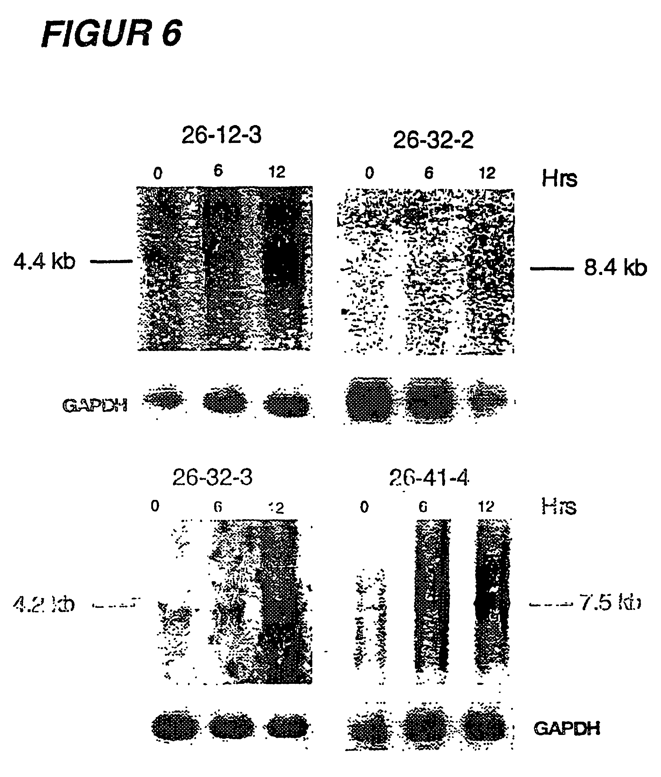

Figur 6 zeigt eine Untersuchung von zellulären, nach IL-3-Entzug induzierten Transkripten. Polyadenylierte RNA (5 µg/Bahn) wurde von FDCP1-Zellen gewonnen, denen IL-3 für 0,6, und 12 Stunden entzogen wurde. Northern-Blots wurden wie in der Legende zu Figur 5 hergestellt und mit ³²P-markierten-5'-Provirus-flankierenden Sequenzen oder GAPDH hybridisiert.

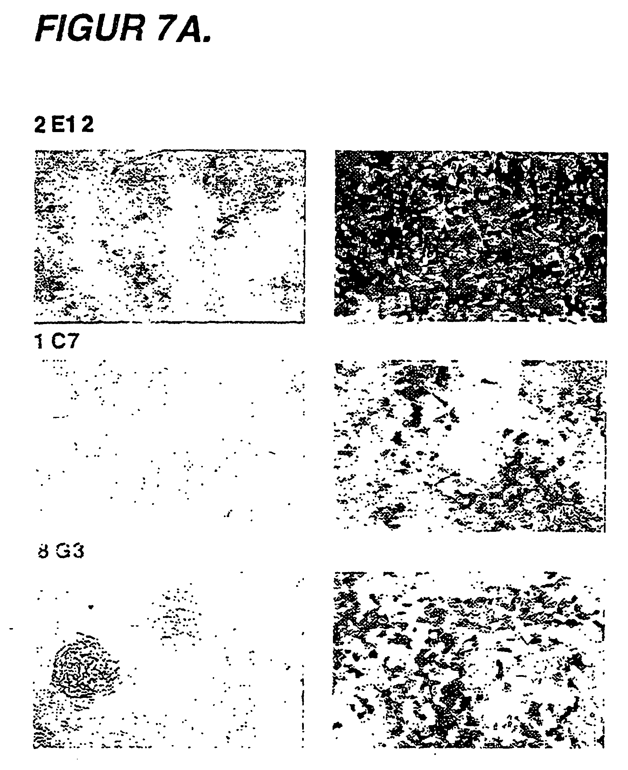

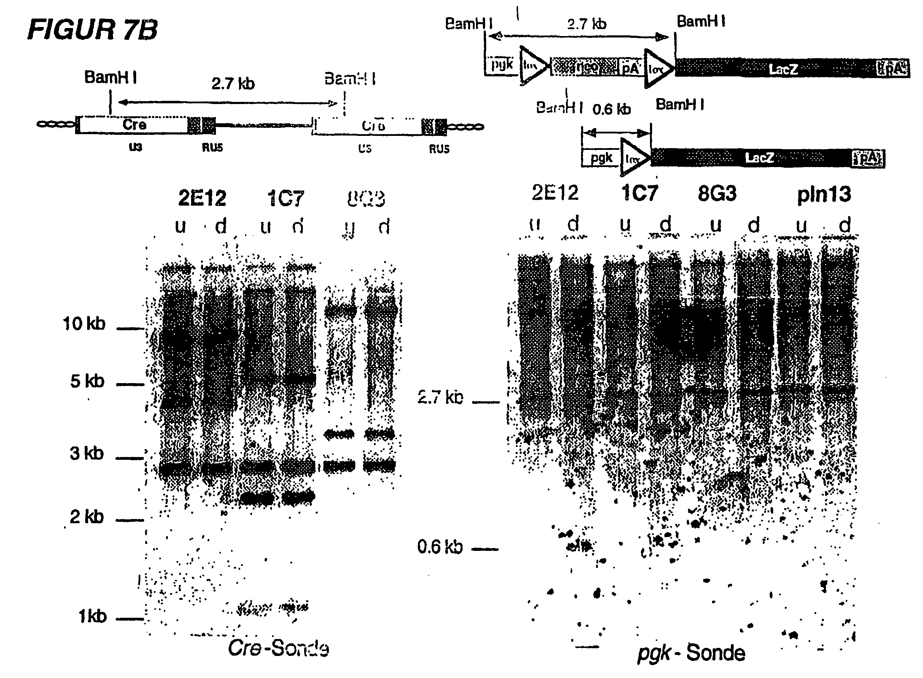

Figur 7 zeigt die differenzierungsregulierte U3-Cre-Genfallenintegrationen in embryonale Stammzellen. 960 Pools zu je 150 Zellen wurden mit dem U3-Cre-Virus infiziert und in G418 selektiert. Differenzierungsinduktion erfolgte über 4 Tage. **(Links)** X-Gal Färbung vor (li.) und nach (re.) Differenzierungsinduktion. **(Rechts)** Southern-Blot-Analyse der regulierten Klone. Die genomische DNA wurde mit BamHI geschnitten und wie in der Legende zu Figur 3 behandelt. Die Hybridisierung erfolgte mit ³²P-markierten Cre- (links) oder pgk- (rechts) spezifischen Sonden. u = undifferenziert; d = differenziert.

Einzelheiten dazu sind den Beispielen zu entnehmen.

Im folgenden soll die Erfindung anhand von Beispielen im einzelnen beschrieben werden, wobei die Beispiele jedoch den Umfang der Erfindung nicht limitieren sollen.

### BEISPIELE

### Beispiel 1

### Herstellung eines Reporterplasmids

Die meisten Bestandteile des Reporterplasmids ppgklxtkneolL-3 (Phosphoglyceratkinasepromotor, loxP-Targetsequenz, Thymidinkinase-Neomycinphosphotransferase-Fusionsprotein, IL-3-Gen) wurden in p-BluescriptII-KS wie folgt zusammengestellt:

Die Sequenzen für die Targetsequenz loxP wurden aus pGEM30 entnommen. Ein loxP-Ort wurde zunächst in den Bluescript Polylinker als ein EcoRI/PstI-Fragment inseriert. Anschließend wurde pgk (Phosphoglyceratkinasepromotor, erhalten von ppgkCAT) in die Xbal/BamHI-Rekombinationsstelle des Polylinkers ligiert und das Thymidinkinase-Neomycinphosphotransferase-Fusionsgen wurde in die stromabwärts gelegene EcoRV-Rekombinationsstelle kloniert. Das IL-3-Gen (als cDNA von Mäusen, aus pc-Multi-CSF) wurde zunächst in die EcoRI-Stelle des Polylinkers von pGEM30 subkloniert, der die loxP-Targetsequenzen flankierte, nachdem zunächst das Plasmid mit ClaI/SalI gespalten wurde und die aufgefüllten Enden wieder ligiert wurden, um eine zusätzliche EcoRI-Stelle zu entfernen. IL-3-loxP wurde dann aus pGEM30 als ein SalI/XhoI-Fragment gewonnen und in den Bluescript Polylinker kloniert. Eine Kopie der Rinderwachstumshormon-Polyadenylierungssequenz (bpa) als XbaI/AvaI-Fragment wurde mit glatten Enden in die ClaI-Stelle stromabwärts von tkneo kloniert. Um ppgklxtkneo-IL-3 zu erhalten, wurde ein Xhol-Fragment, das die gesammelten Sequenzen enthielt, in pSBC2, stromaufwärts einer SV-40-Polyadenylierungsstelle kloniert. Da dieses anfängliche Konstrukt noch eine Translation von IL-3 von dem dicistronischen tkneo-IL-3-Transkript ermöglichte, wurde eine zweite Kopie von bpa mit glatten Enden in die Sall-Stelle von pSBCII kloniert, um das endgültige ppgklxtkneo-IL-3-Reporterplasmid zu erhalten

### Beispiel 2

### Konstruktion einer FDCP1-Reporterzellinie, die zwei selektierbare Reportergene exprimiert, die flankiert sind von loxP-Rekombinationstargets

Die verwendeten FDCP1 hämatopoetischen Vorläuferzellen sind strikt abhängig von IL-3 für ihr Wachstum und initiieren den programmierten Zelltod, wenn dieser Wachstumsfaktor dem Medium entzogen wird.

Die FDCP1-Zellen wurden bei Konzentrationen von 2 x 10⁵ Zellen pro ml in Dulbeccos modifizierten Eagles-Medium (DMEM; Gibco), ergänzt mit 10 % v/v fötalem Rinderserum (High Clone Laboratories, Utah, USA) und 10 ng/ml rekombinanten Mäuse-IL-3 (Sandoz) gezüchtet. Die Agarkulturen waren eine Mischung von gleichen Volumen von doppelstarkem DMEM, ergänzt mit 40 %igem (v/v) fötalem Rinderserum (Hyclone) und 0,6 % (w/v) Bactoagar (Difco) in doppelt destilliertem Wasser. Etwaige Kulturen enthielten 5 µM Gancichlovir (Syntex).

Um eine Zellinie zu erhalten, die sich durch Cre-Rekombinase in eine faktorunabhängige Zelle umwandeln läßt, wurden FDCP1-Zellen mit dem Reporterplasmid ppgklxtkneo-IL-3 elektroporiert. Die Elektroporation wurde unter Verwendung eines BioRad Gene Pulser (BioRad) nach den Instruktionen des Herstellers durchgeführt. Die Rekombinanten wurden in Agarkufturen isoliert, die 0,6 mg/ml G418 enthielten. Die sich entwickelnden Klone wurden nach 10 Tagen isoliert und in Suspensionskulturen wie oben beschrieben expandiert. Das ppgklxtkneo-IL-3 (Figur 3A) besteht aus zwei hintereinander angeordneten selektierbaren Reportergenen, die von einem pgk(Phosphoglyceratkinase)-Promotor transkribiert werden. Das Gen am 5'-Ende kodiert für ein HSV2-Thymidinkinase(Tk)-Neomycinphosphotransferase(neo)-Fusionsprotein und wird von zwei loxP-Rekombinationstargets flankiert. Das 3'-Ende des Gens kodiert für IL-3 aus Mäusen und endet in einer SV40-Polyadenylierungssequenz. Um die IL-3-Translation von dicistronischen Transkripten zu unterdrücken, wurden zwei hintereinander gelegene Kopien der Polyadenylierungssequenz des Rinderwachstumshormons (bpa) stromabwärts von tkneo inseriert. Auf diese Weise konnte man annehmen, daß auch Zellen, die ppgklxtkneo-IL-3 exprimierten, immer noch IL-3-abhängig waren. Da die Cre-Rekombinase typischerweise diejenigen Sequenzen ausschneidet, die von direkten loxP-Wiederholungen flankiert werden, wurde angenommen, daß die Cre-Expression das tkneo-Gen deletiert und dadurch das IL-3-Gen genau stromabwärts des pgk-Promotors plaziert. Als Ergebnis würden die Zellen ihre Neomycinresistenz verlieren und eine Faktorunabhängigkeit durch Synthese von IL-3 erwerben.

Stabile FDCP1-Transformanten wurden in G418 selektiert und fünf klonale Zellinien wurden aus den Agarkulturen isoliert. Eine Zellinie, die ein Einzelkopieplasmid exprimierte, und im weiteren als FLOXIL-3 bezeichnet werden wird, wurde zur weiteren Analyse selektiert.

Zunächst wurde festgestellt, ob die FLOXIL-3-Zellen immer noch für ihr Wachstum von IL-3 abhängig waren, indem 5 x 10⁷ Zellen in einer Dichte von 2 x 10⁵ pro ml in halbfeste (Agar-) Kulturen ohne IL-3 plattiert wurden. Da sich innerhalb von 10 Tagen keine Kolonien entwickelten, wurde angenommen, daß weder eine geringe IL-3-Translation noch spontane Rekombination innerhalb dieser Zellen aufgetreten war.

Um dies weiterhin sicherzustellen, wurden FLOXIL-3 und Eltern-FDCP1-Zellen in Agarkulturen ohne IL-3 für bis zu 24 Stunden vorinkubiert und anschließend durch Gabe von IL-3 gerettet. Beide Zelltypen initierten den programmierten Zelltod mit ähnlichen Kinetiken, was andeutet, daß die Expression von ppgklxtkneo-IL-3 die zelluläre Antwort auf den Faktorentzug nicht veränderte. Außerdem überlebten die meisten Zellen für mehr als 12 Stunden ohne IL-3 und ließen so genügend Zeit für die Expression der Genfallen-transduzierten Sequenzen.

### Beispiel 3

### Herstellung einer Genfalle, die Cre-Rekombinase (U3-Cre) exprimiert und FLOXIL-3-Zellen faktorunabhängig macht

Ein U3-Cre-Genfallen-Vektor wurde aus dem auf MoMuLV basierenden pGgU3Neo(en-)Vektor abgeleitet, indem das neo-Gen in der U3-Region mit der für Cre kodierenden Sequenz, die aus pMCCre abgeleitet wurde, ersetzt wurde. Das Plasmid wurde in Helferzellen transfiziert und die Überstände der Zellinien, die hohe Titer von rekombinanten Viren produzierten, wurden verwendet, um FLOXIL-3-Zellen zu infizieren. Wie schon früher gezeigt, plaziert die Virusreplikation und LTR-vermittelte Duplikation die Sequenzen, die in U3 inseriert sind, genau 30 Nukleotide stromabwärts von der flankierenden zellulären DNA. Dies ermöglicht deren Expression aus Integrationen in transkribierte Gene. Es wurde daher erwartet, daß sich FLOXIL-3-Zellen mit U3-Cre-Integrationen in exprimierten Genen in faktorunabhängige Zellen umwandeln würden. Um auf dieses Ereignis zu selektieren, wurden virusinfizierte FLOXIL-3-Zellen in Agarkulturen ohne IL-3 plattiert. Es wurden mehrere autonom wachsende Kolonien erhalten und in Suspensionskulturen expandiert. Wie von der Expression eines rekombinierten Reporterplasmids zu erwarten war, verloren alle Klone ihre G418-Resistenz und wuchsen ohne IL-3.

Um die Rekombination zu bestätigen, wurden genomische DNAs von fünf autonomen Klonen mit BamHI verdaut und durch Southern Blotting analysiert. Da BamHI innerhalb der 5'-Enden von pgk und IL-3 spaltet, erzeugen nicht rekombinierte FLOXIL-3-Zellen ein internes Fragment von 3,2 kb, das sowohl mit pgk- als auch neo-spezifischen Sonden hybridisiert (Figur 3A). Da dieses Fragment alle Sequenzen enthält, die von loxP flankiert sind, inklusive tkneo, sollte seine Deletion mit einer Reduktion in der Größe von 2,6 kb verbunden sein. Tatsächlich produzierten alle Klone ein 0,6 kb-Restriktionsfragment, das nicht mit neo hybridisierte, was andeutete, daß eine Rekombination stattgefunden hatte (Figur 3B).

Außerdem exprimierten alle Klone Zell-Provirus-Fusionstranskripte, wenn sie durch Northern Blotting analysiert wurden, was andeutete, daß die Cre-Rekombinase von aktiven zellulären Promotoren exprimiert wurde.

Auf diese Weise werden FLOXIL-3-Zellen erhalten, die ein Genfallen-Konstrukt enthalten, das es ermöglicht, diejenigen Gene zu isolieren und identifizieren, die für den programmierten Zelltod verantwortlich sind.

### Beispiel 4

### Herstellung einer U3-Cre/FLOXIL-3-Integrationsbank und Isolierung von transient exprimierten Gensequenzen

Eine Integrationsbank, bestehend aus 2 x 10⁶ unabhängigen proviralen Integrationen, wurde durch Infektion von FLOXIL-3-Zellen mit U3-Cre-Retroviren hergestellt. Die infizierten Zellen wurden zunächst in G418 vorselektiert, um sämtliche Integrationen in exprimierte Gene zu eliminieren. Figur 4 zeigt, daß dieses Ziel nach 16-tägiger Selektion erreicht wurde. Nach Ausplattierung einer simplen Repräsentation der Integrationsbank in Agarkulturen ohne IL-3 konnten insgesamt 110 autonome Klone isoliert und expandiert werden. Southern-Blot-Untersuchungen haben gezeigt, daß sämtliche Klone eine Provirusintegration aufwiesen und durchweg rekombinierte Reporterplasmide enthielten (Figur 4).

Northern-Blot-Untersuchungen mit mRNA von 11 unabhängigen autonomen Klonen zeigen, daß in 9 Klonen Zell-Provirus-Fusionstranskripte entweder nur sehr schwach oder gar nicht exprimiert waren (Figur 5, Bahnen 1,3,7,9,11,13,17,19, 21). Dies besagt, daß die Cre-Rekombinase nur transient stark genug exprimiert war, um eine Rekombination des Reporterplasmids zu verursachen. Ferner waren über 50% dieser Fusionstrankripte durch Serumentzug induzierbar, was andeutet, daß die von der Genfalle unterbrochenen Gene mit einem apoptotischen Programm- oder Wachstumsarrest verbunden sind (Figur 5).

Schließlich wurden die nach bekannten Verfahren klonierten 5'-Provirus-flankierenden Sequenzen (von Melchner et al., 1992) mit der mRNA von Wildtyp FDCP1-Zellen auf Northern Blots hybridisiert. Die Mehrzahl der untersuchten Proben hybridisierte mit IL-3-induzierten, zellulären Transkripten (Figur 6). Ob diese Gene die Selbstmordmaschinerie direkt kontrollieren oder Teil anderer Mechanismen sind, die in Folge des programmierten Zelltods aktiviert werden, muß noch festgestellt werden. Auf jeden Fall erlaubt die Charakterisierung der zellulären Transkripte, die durch den IL-3-Entzug induziert werden, weitere Einblicke in die komplexen molekularen Veränderungen, die während des programmierten Zelltods auftreten.

Die weitere genaue Identifizierung und Isolierung der entdeckten Gene kann mit den bekannten Verfahren durchgeführt werden, so z.B. 3'RACE oder der Erstellung einer Genbibliothek und.Klonierung und Sequenzierung der entdeckten Gene.

### Beispiel 5

### Konstruktion einer ES-Reporterzellinie

Um eine ES-Zellinie zu erhalten, die einem permanenten selektierbaren Reporterwechsel durch Cre-Rekombinase zugänglich war, wurden D3-Zellen mit dem Reporterkonstrukt ppgklxneolacZ elektroporiert, in dem zwei hintereinander angeordnete Reportergene von einem Maus-Phosphoglyceratkinase(pgk)-Promotor transkribiertä werden. Das Reporterkonstrukt ppgklxneolacZ besteht aus dem Phosphoglyceratkinasepromotor, den loxP-Targetsequenzen, dem Neomycinphosphotransferase-Gen und einem Gen für lacZ. Seine Herstellung erfolgt analog der oben beschriebenen Herstellung des Reporterplasmids ppgklxtkneo-IL-3.

Das 5'-Gen kodiert für Neomycinphosphotransferase und wird von zwei loxP-Rekombinationstargets in identischer Orientierung flankiert. Das 3'-Gen kodiert für β-Galaktosidase und endet in einer SV40-Polyadenylierungssequenz. Um genomische Transkripte zu unterdrücken, die in SV40-PolyA enden und auf diese Weise auch die Translatiön von lacZ zu unterdrücken, wurden zwei hintereinanderliegende Kopien von Rinderwachstumshormon-Polyadenylierungssequenz (bpa) stromabwärts der Neomycinphosphotransferase inseriert. Dementsprechend sollten ppgklxneolacZ-exprimierende Zellen einen Neomycinphosphotransferase-resistenten und lacZ-negativen Phanotyp aufweisen. Im Falle einer Cre-Expression würde die Rekombinase jedoch das neo-Gen ausschneiden und die lacZ-kodierende Sequenz unmittelbar stromabwärts vom pgk-Promotor plazieren. Im Ergebnis verlieren die Zellen, die rekombinierte Reporterplasmide exprimieren, ihre Neomycinresistenz und färben sich positiv mit X-Gal (lacZ⁺).

Verschiedene ppgklxneolacZ-exprimierende ES-Zellinien wurden in G418 isoliert und individuell auf Hintergrund-X-Gal-Färbung getestet. 4 von 10 Zellinien färbten sich regelmäßig nicht mit X-Gal, sogar nach langen Kulturperioden. Wenn man diese Zellinien dazu induzierte, in Kulturen zu differenzieren, die kein LIF (Leukemie Inhibitory Factor) oder Nährschichten enthielten, färbten sich lacZ⁻-Zellen weiterhin negativ, was andeutete, daß der lacZ-Phänotyp stabil ist. Eine Zellinie, die eine einzelne Kopie von ppgklxneolacZ exprimierte, wurde für eine weitere Analyse selektiert. Sie wird im weiteren als pln13 bezeichnet.

Um festzustellen, ob die Cre-Rekombinase den lacZ-Phänotyp von pln13 in lacZ⁺ umwandelt, wurden die Zellen mit Cre-exprimierendem Plasmid pMC-Cre transfiziert. Nicht selektierte Kolonien wurden nach 10 Tagen gepickt und Aliquots wurden mit X-Gal gefärbt. Genomische DNA, die aus verschiedenen lacZ⁺-Zellen extrahiert wurde, wurde mit BamHI gespalten und durch Southem Blotting analysiert. In jedem Fall war der lacZ-Phanotyp mit einer 2 kb-Deletion assoziiert, was andeutete, daß das Reporterplasmid einer ortsspezifischen Rekombination unterworfen war.

### Beispiel 6

### Expression von β-Galaktosidase in pln13-Zellen durch U3-Cre-Integration

5 x 10⁴ pIn13-Zellen wurden mit U3-Cre-Virus, hergestellt wie oben beschrieben, bei einer MOI = 1 infiziert. Nach einer Inkubation von 72 Stunden wurden die Zellen in 480 Pools geteilt und in Mikrotiterplatten ausgesät. Nach 48 Stunden wurden Aliquots mit X-Gal gefärbt, um Integrationen in aktive Gene zu identifizieren. Von 20 Pools, die lacZ⁺-Zellen enthielten, wurden 3 in klonaler Dichte ausgesät. 4 positive Klone wurden durch Selektion isoliert und durch Southern-Blotting analysiert. Alle Klone enthielten 1 bis 2 Proviren, die mit einem MOI = 1 übereinstimmten und enthielten rekombinierte Reporterplasmide. Jeder Klon exprimierte außerdem Zell-Provirus-Fusionstranskripte, deren Größe erwartungsgemäß um 100 bis 500 nt variierte.

Auf diese Weise vermitteln U3-Cre-Genfallen-Retroviren effektiv ortsspezifische Rekombinationen aufgrund ihrer Integration in exprimierte Gene.

### Beispiel 7

### Isolierung von Klonen mit induzierbarer U3-Cre-Provirusintegration

Um festzustellen, ob die U3-Cre/loxP-Strategie geeignet ist, um Gene zu isolieren, die durch Differenzierung induzierbar sind, wurden 1,5 x 10⁵ pln13-Zellen in die Vertiefungen von 10 Mikrotiterplatten bei Konzentrationen von 150 Zellen pro Vertiefung ausgesät und mit U3-Cre-Virus bei ungefähr MOI = 1 infiziert. Um Integrationen in aktive Gene zu eliminieren, wurden Zellen auf G418 für 5 Tage selektiert. Anschließend wurden Aliquots jeder Vertiefung in Abwesenheit von sowohl LIF (Leukemia Inhibitory Factor) oder MEF (Mouse Embryonic Fibroblasts) zum Differenzieren induziert und mit X-Gal gefärbt. 44 von 960 Vertiefungen färbten sich bei diesem Test positiv. Nach einem weiteren Test waren 9 Pools lacZ⁺ sowohl in Gegenwart als auch in Abwesenheit von LIF und MEF, während 35 Pools zu lacZ⁺ nur infolge von Differenzierungen wechselten, was andeutete, daß U3-Cre-Integrationen in den induzierten Genen aufgetreten waren.

Figur 7 zeigt beispielhaft drei isolierte Klone mit U3-Cre-Provirusintegrationen in differenzierungsspezifische Gene. Die Induzierbarkeit der Gene wird durch den phänotypischen Wechsel von LacZ(-) (Figur 7A, undifferenzierte Zellen, oben li.) zu LacZ(+) (Figur 7A, differenzierte Zellen, oben re.) belegt. Die Southern-Blots beweisen, daß diesem Wechsel die ortspezifische Rekombination des Reporterplasmids zugrunde liegt, die von U3-Cre-Provirusintegrationen in induzierbare Differenzierungsgene verursacht wurde (Figur 7B).

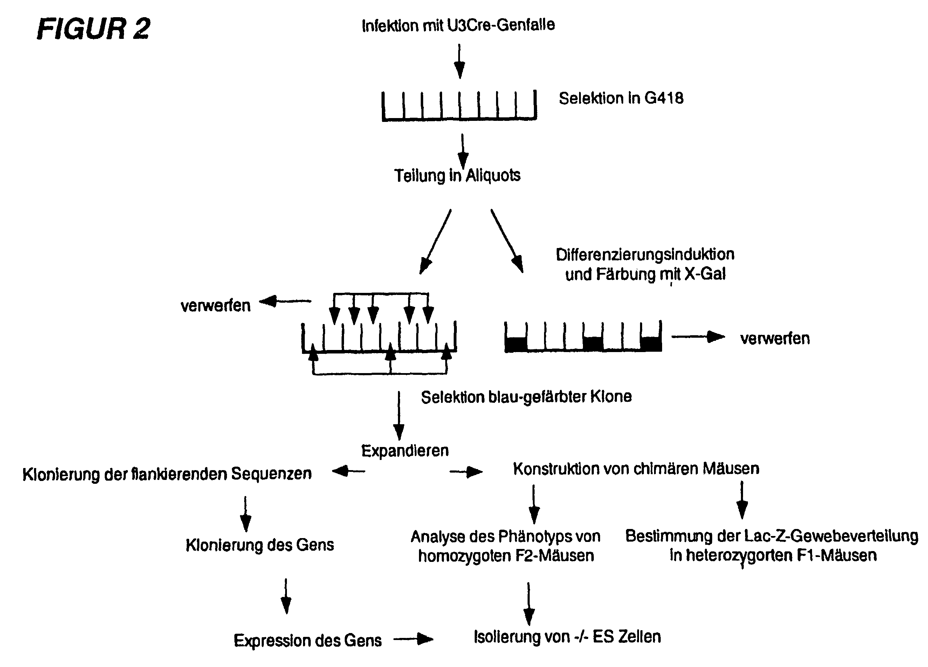

In Figur 2 ist eine Übersicht über eine derartige Isolierung gezeigt. Im ersten Schritt wird mit U3-Cre-Genfallen-Vektor infiziert und auf G418 selektiert. Die erhaltenen Kolonien werden in Aliquots unterteilt, zum Differenzieren induziert und mit X-Gal gefärbt. Nur die gefärbten Klone werden weiter selektiert, expandiert und zur Konstruktion von chimären Mäusen verwendet. Diese Mäuse können wiederum verwendet werden, um die lacZ-Verteilung im Gewebe zu bestimmen oder den Phänotyp von homozyoten F2-Mäusen zu bestimmen und -/-ES-Zellen zu isolieren.

Die entsprechenden zellulären Gene können von den bekannten Genfallensequenzen durch bekannte Verfahren kloniert werden.

Auf diese Weise ermöglicht das Genfallen-Konstrukt gemäß der vorliegenden Erfindung, Gene, insbesondere nur transient exprimierte Gene, zu isolieren und/oder zu identifizieren.

### Literaturliste

Coffin, J. M., Stoye, J. P., and Frankel, W. N. (1989). Genetics of endogenous murine leukemia viruses. Ann. N Y Acad. Sci. *567*, 39-49.

Goff, S. P. (1990). Integration of retroviral DNA into the genome of the infected cell. Cancer Cells, 172-178.

Gridley, T., Soriano, O., and Jaenisch, R. (1987). Insertional mutagenesis in mice. Trends Genet. *3*, 162-166.

Hill, D. H. P., and Wurst, W. (1993). Mutagenic strategies to dissect mammalian development. Curr. Topics. Dev. Biol. *28*, 181-206.

Hill, D. H. P., and Wurst, W. (1993). Screening for pattern formation in mouse using gene trap technology. Methods Enzym. *255*, 663-681.

Muthuchamy, M., Pajak, L., Howles, P., Doetschman, T., and Wieczorek, D. (1993). Developmental analysis of tropomyosin gene expression in embryonic stem cells and mouse embryos. Mol. Cell. Biol. *13*, 3311-3323.

Rappolee, D. A., Brenner, C. A., Schultz, R, Mark, D., and Werb, Z. (1988). Developmental expression of PDGF, TGF-a and TGF-b in preimplantation mouse embryos. Science *241*, 1823-1825.

Reddy, S., Rayburn, H., von Melchner, H., and Ruley, H. E. (1992). Fluorescence activated sorting of totipotent embryonic stem cells expressing developmentally regulated lacZ fusion genes. Proc. Natl. Acad. Sci. USA *89*, 6721-6725.

Rogers, M. B., Hosler, B. A., and Gudas, L. J. (1991). Specific expression of a retinoid acid-regulated, zinc-finger gene, Rex-1, in preimplanation embryos, trophoblast and spermatocytes. Development *113*, 815-824.

Sandmeyer, S. B., Hansen, L. J., and Chalker, D. L. (1990). Integration specificity of retrotransposons and retroviruses. Ann. Rev. Genet. *24*, 491-518.

Scholer, H. R., Dressler, G. R., Balling, R., Rohdewohld, H., and Gruss, P. (1990). Oct-4: a germline-specific transcription factor mapping to the mouse t-complex. EMBO J *9*, 2185-2195.

Sharpe, N. G., Williams, D. G., and Latchman, D. S. (1990). Regulated expression of the small nuclear ribonucleoprotein particle protein SmN in embryonic stem cell differentiation. Mol. Cell. Biol. *10*, 6817-6820.

Skarnes, W. C., Auerbach, B. A., and Joyner, A. L. (1992). A gene trap approach in mouse embryonic stem cells: the lacZ reporter is activated by splicing, reflects endogenous gene expression, and is mutagenic in mice. Genes Dev. *6*, 903-918.

Varmus, H. (1988). Retroviruses. Science *240*, 1427-1435.

von Melchner, H., DeGregori, J. V., Rayburn, H., Reddy, S., Friedel, C., and Ruley, H. E. (1992). Selective disruption of genes expressed in totipotent embryonal stem cells. Genes Dev *6*, 919-927.

von Melchner, H., and Ruley, H. E. (1995). Functional analysis of the mammalian genome by gene entrapment. In Functional Analysis of the Human Genome, F. Farzane and D. N. Cooper, eds. (Oxford: Bios Scientific Publishers). 109-124.

Withers-Ward, E. S., Kitamura, Y., Barnes, J. P., and Coffin, J. M. (1994). Distribution of targets for avian retrovirus DNA integration in vivo. Genes Dev. *8*, 1473-1487.

Jaenisch, R., Breindl, M., Harbers, K., Jahner, D., and Lohler, J. (1985). Retroviruses and insertional mutagenesis. Cold Spring Harb. Symp. Quant. Biol. *50*, 439-445.

Friedrich, G., and Soriano, P. (1991). Promoter traps in embryonic stem cells: a genetic screen to identify and mutate developmental genes in mice. Genes Dev. *5*,1513-1523.

DeGregori, J., Russ, A., von Melchner, H., Rayburn, H., Priyaranjan, P., Jenkins, N. A., Copeland, N. G., and Ruley, H. E. (1994). A murine homolog of the yeast RNA1 gene is required for postimplantation development. Genes Dev. *8*, 265-276.

## Patentansprüche

1. Genfallen-Konstrukt, enthaltend ein erstes Reportergen, das nach Aktivierung ein zweites Reportergen aktivieren kann, wobei
das erste Reportergen für eine Rekombinase kodiert, das zweite Reportergen einen Proteinfaktor kodiert und das zweite Reportergen dadurch aktiviert wird, daß die Rekombinase ein vor dem zweiten Reportergen gelegenes DNA-Fragment deletiert und das zweite Reportergen somit stromabwärts von einem Promotor unter dessen Kontrolle plaziert.

2. Genfallen-Konstrukt gemäß Anspruch 1, **dadurch gekennzeichnet, daß** die Rekombinase Cre ist.

3. Genfallen-Konstrukt gemäß Anspruch 1, **dadurch gekennzeichnet, daß** die Rekombinase Flp ist.

4. Genfallen-Konstrukt gemäß Anspruch 1, **dadurch gekennzeichnet, daß** das zweite Reportergen für IL-3 kodiert.

5. Genfallen-Konstrukt gemäß Anspruch 1, **dadurch gekennzeichnet, daß** das zweite Reportergen für lacZ kodiert.

6. Genfallen-Konstrukt gemäß Anspruch 1, **dadurch gekennzeichnet, daß** das deletierte DNA-Fragment ein Antibiotika-Resistenzgen ist.

7. Genfallen-Konstrukt gemäß Anspruch 6, **dadurch gekennzeichnet, daß** das deletierte DNA-Fragment für ein Thymidin-Kinase-Neomycinphosphotransferase-Fusionsprotein kodiert.

8. Genfallen-Konstrukt gemäß Anspruch 7, **dadurch gekennzeichnet, daß** das deletierte DNA-Fragment für eine Neomycinphosphotransferase kodiert.

9. Genfallen-Konstrukt gemäß Anspruch 6, 7 oder 8, **dadurch gekennzeichnet, daß** der Promotor der Phosphoglyceratkinase (pgk)-Promotor ist.

10. Genfallen-Konstrukt gemäß einem oder mehreren der Ansprüche 1 bis 9, **dadurch gekennzeichnet, daß** das deletierte DNA-Fragment von Targetsequenzen für die Rekombinase flankiert ist.

11. Genfallen-Konstrukt gemäß Anspruch 10, **dadurch gekennzeichnet, daß** die Targetsequenz loxP ist.

12. Genfallen-Konstrukt gemäß Anspruch 10, **dadurch gekennzeichnet, daß** die Targetsequenz frt ist.

13. Genfallen-Konstrukt gemäß Anspruch 10, 11 oder 12, **dadurch gekennzeichnet, daß** sich die Targetsequenzen in der U3 und/oder U5-Region befinden.

14. Verwendung des Genfallen-Konstrukts gemäß einem oder mehreren der vorstehenden Ansprüche für eine Identifizierung und/oder Isolierung von Genen, insbesondere transienten Genen.

15. Verwendung gemäß Anspruch 14 für eine Identifizierung und/oder Isolierung von den programmierten Zelltod, die DNA-Reparatur, die Differenzierung und/oder den Zellzyklus kontrollierenden Genen.

16. Zelle enthaltend ein Genfallen-Konstrukt gemäß einem oder mehreren der Ansprüche 1 bis 13, wobei es sich nicht um eine totipotente menschliche Stammzelle handelt.

17. Zelle gemäß Anspruch 16, **dadurch gekennzeichnet, dass** es eine Säugetierzelle ist.

18. Säugetierzelle gemäß Anspruch 17, **dadurch gekennzeichnet, daß** sie von IL-3 abhängig ist, daß das erste Reportergen für Cre-Rekombinase kodiert, daß die Cre-Rekombinase ein vor dem zweiten Reportergen gelegenes DNA-Fragment deletieren kann, daß das deletierte Fragment für ein Thymidinkinase-Neomycinphosphotransferase-Fusionsprotein (TKNeoPT) kodiert und von loxP-Targetsequenzen flankiert ist, daß das zweite Reportergen für IL-3 kodiert und nach Deletion des TKNeoPT-Gens stromabwärts des Phosphoglyceratkinase-Promotors zu liegen kommt.

19. Säugetierzelle gemäß Anspruch 18, **dadurch gekennzeichnet, daß** es eine hämatopoetische FDCP-1-Zelle oder eine totipotente Stammzelle ist.

20. Verwendung der Säugetierzelle gemäß Anspruch 17 für eine Identifizierung und/oder Isolierung von Genen, insbesondere transienten Genen.

21. Verwendung der Säugetierzelle gemäß Anspruch 17 oder 18 oder 19 für eine Identifizierung und/oder Isolierung von den programmierten Zelltod, die DNA-Reparatur, die Differenzierung und/oder den Zellzyklus kontrollierenden Genen.

22. Vektor, enthaltend das Genfallen-Konstrukt gemäß einem oder mehreren der Ansprüche 1 bis 13.

23. Kit zur Identifizierung und/oder Isolierung von Genen, insbesondere transienten Genen, enthaltend mindestens ein Genfallen-Konstrukt gemäß einem oder ' mehreren der Ansprüche 1 bis 13 und/oder einen Vektor nach Anspruch 22.

24. Verfahren zur Identifizierung und/oder Isolierung von Genen das die folgenden Schritte enthält:
- Einbau eines Genfallen-Konstrukts nach einem der Ansprüche 1 bis 13 in eine geeignete Zelle; wobei es sich nicht um eine totipotente menschliche Stammzelle handelt;
- Selektion auf Zellen, bei denen das erste Reportergen in ein Gen, vorzugsweise nicht aktives Gen, eingebaut ist;
- Aktivierung des ersten Reportergens, vorzugsweise durch Initiation der Transkription des Gens, woraufhin das zweite Reportergen aktiviert wird; und
- Identifizierung und/oder Isolierung des Gens, in das das erste Reportergen eingebaut ist.

25. Verfahren gemäß Anspruch 24 zur Identifizierung und/oder Isolierung von transienten Genen.

26. Verfahren gemäß Anspruch 24, **dadurch gekennzeichnet, daß**
- ein Genfallen-Konstrukt gemäß Anspruch 2 als erstes Reportergen das Cre-Rekombinase-Gen enthält, daß die Cre-Rekombinase ein stromabwärts vom zweiten Reportergen gelegenes Thymidinkinase-Neomycinphosphotransferase-Gen deletiert, wodurch das zweite Reportergen, das für IL-3 kodiert, neben einem Phosphoglyceratkinase-Promotor zu liegen kommt,
- das Genfallen-Konstrukt in eine IL-3-abhängige FDCP-1-Zelle eingeschleust wird,
- durch Anzucht ein Neomycin-Medium auf Zellen selektiert wird, die Cre-Rekombinase nicht produzieren,
- diese selektierten Zellen in IL-3-freiem Medium gezüchtet werden, so daß für den programmierten Zelltod verantwortliche Gene aktiviert werden,
- daß die überlebenden Zellen selektioniert werden und
- die für den programmierten Zelltod verantwortlichen Gene mit den bekannten Verfahren isoliert werden.

27. Verfahren gemäß Anspruch 24, **dadurch gekennzeichnet, daß**
- ein Genfallen-Konstrukt gemäß Anspruch 2 als erstes Reportergen das Cre-Rekombinase-Gen enthält, daß die Cre-Rekombinase ein stromabwärts vom zweiten Reportergen gelegenes Neomycinphosphotransferase-Gen deletiert, wodurch das zweite Reportergen, das für lacZ kodiert, neben einem Phosphoglyceratkinase-Promotor zu liegen kommt,
- das Genfallen-Konstrukt in eine totipotente nicht-menschliche embryonale Stammzelle eingeschleust wird,
- durch Anzucht auf G418 auf Zellen selektiert wird, die Cre-Rekombinase nicht produzieren,
- diese selektiven Zellen zum Differenzieren induziert werden, so daß differenzierungsspezifische Gene aktiviert werden,
- diese Zellen isoliert werden,
- diese Zellen in die Keimbahn eines nicht menschlichen Säugetiers eingeführt werden und dort für die Bildung von Geweben verantwortlich sind und
- die differenzierzungsspezifischen Gene mit den bekannten Verfahren isoliert werden.

## Claims

1. Gene-trapping construct, containing a first reporter gene, which after activation can activate a second reporter gene, wherein the first reporter gene codes for a recombinase, the second reporter gene codes for a protein factor and the second reporter gene is activated thereby that the recombinase deletes a DNA fragment located before the second reporter gene and in that way places the second reporter gene downstream from a promotor under its control.

2. Gene-trapping construct according to Claim 1, characterized thereby that the recombinase is Cre.

3. Gene-trapping construct according to Claim 1, characterized thereby that the recombinase is Flp.

4. Gene-trapping construct according to Claim 1, characterized thereby that the second reporter gene codes for IL-3.

5. Gene-trapping construct according to Claim 1, characterized thereby that the second reporter gene codes for LacZ.

6. Gene-trapping construct according to Claim 1, characterized thereby that the deleted fragment is an antibiotic-resistance gene.

7. Gene-trapping construct according to Claim 6, characterized thereby that the deleted DNA fragment codes for a thymidine kinase-neomycin phosphotransferase fusion protein.

8. Gene-trapping construct according to Claim 7, characterized thereby that the deleted DNA fragment codes for a neomycin phosphotransferase.

9. Gene-trapping construct according to Claim 6, 7 or 8, characterized thereby that the promotor is the phosphoglycerate kinase (pgk) promotor.

10. Gene-trapping construct according to one or more of the Claims 1 to 9, characterized thereby that the deleted DNA fragment is flanked by target sequences for the recombinase.

11. Gene-trapping construct according to Claim 10, characterized thereby that the target sequence is IoxP.

12. Gene-trapping construct according to Claim 10, characterized thereby that the target sequence is frt.

13. Gene-trapping construct according to Claim 10, 11 or 12, characterized thereby that the target sequences are located in the U3 and/or the U5 region.

14. Use of the gene-trapping construct according to one or more of the foregoing Claims for identification and/or isolation of genes, in particular transient genes.

15. Use according to Claim 14 for identification and/or isolation of genes controlling programmed cell death, DNA repair, differentiation and/or the cell cycle.

16. Cell containing a gene-trapping construct according to one or more of Claims 1 to 13, wherein the cell is not a totipotent human stem cell.

17. Cell according to Claim 16, characterized thereby that the cell is a mammalian cell.

18. Mammalian cell according to Claim 17, characterized thereby that it is dependent on IL-3, that the first reporter gene codes for Cre recombinase, that the Cre recombinase can delete a DNA fragment located before the second reporter gene, that the deleted fragment codes for a thymidine kinase-neomycin transferase fusion protein (TKNeoPT) and is flanked by IoxP target sequences, that the second reporter gene codes for IL-3 and following deletion of the TKNeoPT gene comes to lie downstream of the phosphoglycerate kinase promotor.

19. Mammalian cell according to Claim 18, characterized thereby that it is a haemopoietic FDCP-1 cell or a totipotent stem cell.

20. Use of the mammalian cell according to Claim 17 for identification and/or isolation of genes, in particular transient genes.

21. Use of the mammalian cell according to Claim 17, 18 or 19 for identification and/or isolation of the genes controlling programmed cell death, DNA repair, differentiation and/or the cell cycle.

22. Vector containing the gene-trapping construct according to one or more of Claims 1 to 13.

23. Kit for identification and/or isolation of genes, in particular transient genes, containing at least one gene-trapping construct according to Claims 1 to 13 and/or a vector according to Claim 22.

24. Process for the identification and/or isolation of genes comprising the following steps:
• Introduction of a gene-trapping construct according to one of the Claims 1 to 13 into a suitable cell which is not a totipotent human stem cell;
• Selection of cells in which the first reporter gene is incorporated in a gene, preferably an inactive gene;
• Activation of the first reporter gene, preferably through initiation of transcription of the gene, in consequence of which the second reporter gene is activated; and
• Identification and/or isolation of the gene in which the first reporter gene is incorporated.

25. Process according to Claim 24 for identification and/or isolation of transient genes.

26. Process according to Claim 24, characterized thereby that
• a gene-trapping construct according to Claim 2 contains as first reporter gene the Cre recombinase gene, that the Cre recombinase deletes a thymidine kinase-neomycin phosphotransferase gene situated downstream from the second reporter gene, whereby the second reporter gene, which codes for IL-3, comes under the control of a phosphoglycerate kinase promotor,
• the gene-trapping construct is inserted into an IL-3-dependent FDCP-1 cell,
• through culture in a neomycin medium cells which do not produce Cre recombinase are selected,
• these selected cells are cultivated in IL-3-free medium so that the genes responsible for programmed cell death are activated,
• that the surviving cells are selected, and
• the genes responsible for programmed cell death are isolated by the customary procedures.

27. Process according to Claim 24, characterized thereby that
• a gene-trapping construct according to Claim 2 contains the Cre recombinase gene as first reporter gene that the Cre recombinase deletes a neomycin phosphotransferase gene located downstream from the second reporter gene, whereby the second reporter gene, which codes for lacZ, comes under the control of a phosphoglycerate kinase promotor,
• the gene-trapping construct is inserted into a totipotent non-human embryonic stem cell,
• through culture in G418, cells which do not produce Cre recombinase are selected,
• these selected cells are induced to differentiate, so that differentiation-specific genes are activated,
• these cells are isolated,
• these cells are introduced into the germ line of a non-human mammal and are there responsible for the formation of tissues and
• the differentiation-specific genes are isolated by the customary procedures.

## Revendications

1. Construction formant piège à gènes, contenant un premier gène reporter qui peut activer un deuxième gène reporter après activation, tandis que le premier gène reporter code pour une recombinase, le deuxième gène reporter code pour un facteur protéique, et le deuxième gène reporter est activé par délétion par la recombinase d'un fragment d'ADN situé devant le deuxième gène reporter et le deuxième gène reporter est ainsi placé en aval d'un promoteur sous son contrôle.

2. Construction formant piège à gènes selon la revendication 1, **caractérisée en ce que** la recombinase est Cre.

3. Construction formant piège à gènes selon la revendication 1, **caractérisée en ce que** la recombinase est Flp.

4. Construction formant piège à gènes selon la revendication 1, **caractérisée en ce que** le deuxième gène reporter code pour IL-3.

5. Construction formant piège à gènes selon la revendication 1, **caractérisée en ce que** le deuxième gène reporter code pour lacZ.

6. Construction formant piège à gènes selon la revendication 1, **caractérisée en ce que** le fragment d'ADN délété est un gène de résistance aux antibiotiques.

7. Construction formant piège à gènes selon la revendication 6, **caractérisée en ce que** le fragment d'ADN délété code pour une protéine de fusion thymidine-kinase-néomycinephosphotransférase.

8. Construction formant piège à gènes selon la revendication 7, **caractérisée en ce que** le fragment d'ADN délété code pour une néomycinephosphotransférase.

9. Construction formant piège à gènes selon la revendication 6, 7 ou 8, **caractérisée en ce que** le promoteur est le promoteur de phosphoglycérate-kinase (pgk).

10. Construction formant piège à gènes selon l'une ou plusieurs des revendications 1 à 9, **caractérisée en ce que** le fragment d'ADN délété est flanqué par des séquences cible pour la recombinase.

11. Construction formant piège à gènes selon la revendication 10, **caractérisée en ce que** la séquence cible est loxP.

12. Construction formant piège à gènes selon la revendication 10, **caractérisée en ce que** la séquence cible est frt.

13. Construction formant piège à gènes selon la revendication 10, 11 ou 12, **caractérisée en ce que** les séquences cible se trouvent dans la région U3 et/ ou U5.

14. Utilisation de la construction formant piège à gènes selon l'une ou plusieurs des revendications précédentes pour une identification et/ ou un isolement de gènes, en particulier de gènes transitoires.

15. Utilisation selon la revendication 14 pour une identification et/ou un isolement de gènes contrôlant la mort cellulaire programmée, la réparation d'ADN, la différenciation et/ ou le cycle cellulaire.

16. Cellule contenant une construction formant piège à gènes selon l'une ou plusieurs des revendications 1 à 13, pour lesquelles il ne s'agit pas d'une cellule souche humaine totipotente.

17. Cellule selon la revendication 16, **caractérisée en ce qu'**elle est une cellule mammalienne.

18. Cellule mammalienne selon la revendication 17, **caractérisée en ce qu'**elle est dépendante de IL-3 que le premier gène reporter code pour la Cre-recombinase, que la Cre-recombinase peut déléter un fragment d'ADN situé avant le deuxième gène reporter, que le fragment délété code pour une protéine de fusion thymidinekinase-néomycinephosphotransférase (TKNeoPT) et est flanqué par des séquences cible loxP, que le deuxième gène reporter code pour IL-3 et après délétion du gène TKNeoPT se trouve situé en aval du promoteur de phosphoglycérate-kinase.

19. Cellule mammalienne selon la revendication 18, **caractérisée en ce qu'**elle est une cellule FDCP-1 hématopoïétique ou une cellule souche totipotente.

20. Utilisation de la cellule mammalienne selon la revendication 17 pour une identification et/ou un isolement de gènes, en particulier de gènes transitoires.

21. Utilisation de la cellule mammalienne selon la revendication 17 ou 18 ou 19 pour une identification et/ou un isolement de gènes contrôlant la mort cellulaire programmée, la réparation d'ADN, la différenciation et/ou le cycle cellulaire.

22. Vecteur, contenant la construction formant piège à gènes selon l'une ou plusieurs des revendications 1 à 13.

23. Kit pour l'identification et/ou l'isolement de gènes, en particulier de gènes transitoires, contenant au moins une construction formant piège à gènes selon l'une ou plusieurs des revendications 1 à 13 et/ou un vecteur selon la revendication 22.

24. Procédé pour l'identification et/ ou l'isolement de gènes, qui comporte les étapes suivantes:
- incorporation d'une construction formant piège à gènes selon l'une des revendications 1 à 13 dans une cellule appropriée, tandis qu'il ne s'agit pas d'une cellule souche humaine totipotente;
- sélection des cellules dans lesquelles le premier gène reporter est incorporé dans un gène, de préférence un gène non-actif;
- activation du premier gène reporter, de préférence par initiation de la transcription du gène, le deuxième gène reporter étant ainsi activé; et
- identification et/ ou isolement du gène, dans lequel le premier gène reporter est incorporé.

25. Procédé selon la revendication 24 pour l'identification et/ou l'isolement de gènes transitoires.

26. Procédé selon la revendication 24, **caractérisé en ce que**
- une construction formant piège à gènes selon la revendication 2 contient comme premier gène reporter le gène de la Cre-recombinase, que la Cre-recombinase délète un gène de thymidinekinase-néomycinephosphotransférase situé en aval du deuxième gène reporter, ce par quoi le deuxième gène reporter, qui code pour IL-3, se trouvé situé à côté d'un promoteur de phophoglycérate-kinase,
- la construction formant piège à gènes est introduite dans une cellule FDCP-1 dépendante de IL-3,
- par culture sur un milieu de néomycine on sélectionne des cellules qui ne produisent pas de Cre-recombinase,
- on cultive ces cellules sélectionnées dans du milieu exempt de IL-3, de manière à activer les gènes responsables de la mort cellulaire programmée,
- on sélectionne les cellules survivantes et
- on isole les cellules responsables de la mort cellulaire programmée avec les procédés connus.

27. Procédé selon la revendication 24, **caractérisé en ce que**
- une construction formant piège à gènes selon la revendication 2 contient comme premier gène reporter le gène de la Cre-recombinase, que la Cre-recombinase délète un gène de néomycinephosphotransférase situé en aval du deuxième gène reporter, ce par quoi le deuxième gène reporter, qui code pour lacZ, vient à se situer à côté d'un promoteur de phophoglycérate-kinase,
- la construction formant piège à gènes est introduite dans une cellule souche embryonnaire non-humaine totipotente,
- par culture sur G418 on sélectionne des cellules qui ne produisent pas de Cre-recombinase,
- on induit ces cellules sélectives à se différencier, de manière à activer des gènes spécifiques de la différenciation,
- on isole ces cellules,
- on introduit ces cellules dans la voie germinative d'un mammifère non-humain et elles y sont responsables de la formation de tissus, et
- on isole les gènes spécifiques pour la différenciation avec les procédés connus.
